# EUROPÄISCHE PATENTSCHRIFT

(11) **EP 0 977 732 B1**
(45) Veröffentlichungstag und Bekanntmachung des Hinweises auf die Patenterteilung: **06.02.2002**
(21) Anmeldenummer: 98917028.7
(22) Anmeldetag: 20.03.1998
(51) Int. Cl.: C07C 305/10, C11D 1/12, B01F 17/00, A61K 7/50

(54) **AMPHIPHILE VERBINDUNGEN MIT MINDESTENS ZWEI HYDROPHILEN UND MINDESTENS ZWEI HYDROPHOBEN GRUPPEN AUF DER BASIS VON DI-, OLIGO- ODER POLYENEN**
AMPHIPHILIC COMPOUNDS WITH AT LEAST TWO HYDROPHILIC AND AT LEAST TWO HYDROPHOBIC GROUPS ON A DI- OLIGO- OR POLYENE BASE
COMPOSES AMPHIPHILES COMPORTANT AU MOINS DEUX GROUPES HYDROPHILES ET AU MOINS DEUX GROUPES HYDROPHOBES A BASE DE DIENES, D'OLIGO-ENES OU DE POLYENES

(30) Priorität: 24.04.1997 DE 19717264
(43) Veröffentlichungstag der Anmeldung: 09.02.2000
(73) Patentinhaber: SASOL Germany GmbH, 22297 Hamburg (DE)
(72) Erfinder: RÜSCH, Klaas, Mark, D-48143 Münster (DE); KWETKAT, Klaus, D-44534 Lünen (DE); WARWEL, Siegfried, D-52074 Aachen (DE)
(74) Vertreter: Schupfner, Gerhard D., Dr. Dipl.-Chem.
(86) Internationale Anmeldenummer: EP9801633
(87) Internationale Veröffentlichungsnummer: WO9847865

(56) Entgegenhaltungen:
- WO-A-96/16033
- WO-A-97/09304

## Beschreibung

Die Erfindung betrifft amphiphile Verbindungen mit mindestens zwei hydrophilen und mindestens zwei hydrophoben Gruppen auf der Basis von Di-, Oligo- oder Polyenen, ein Verfahren zu ihrer Herstellung und ihre Verwendung als Dispergatoren, Emulgatoren, Demulgatoren, als Hilfsmittel bei der Erzgewinnung, Metallbearbeitung, Oberflächenveredelung, Kunststoffherstellung oder Kunststoffverarbeitung, als Hilfsmittel bei der Ausbringung von Pflanzenbehandlungsmitteln, als Hilfsmittel bei medizinischen Anwendungen, als Textilhilfsmittel, als Hilfsmittel für das Reinigen und Waschen von Textilien, als Hilfsmittel für das Reinigen von harten Oberflächen und als Hilfsmittel für das Reinigen und Waschen von Haut und Haar.

Als amphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil.

Bei den amphiphilen Substanzen besteht aus ökologischen Gründen, z. B. bezüglich der Verringerung des Verpackungs- und Transportaufwandes die Notwendigkeit, immer größere Wirkung pro Masse an eingesetzter Substanz zu erzielen. Da eine Optimierung durch Mischung von amphiphilen Substanzen nur sehr begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Ober- und Grenzflächenspannungen gefunden werden, um die Einsatzmengen an Wirksubstanz deutlich reduzieren zu können.

Erste Lösungsansätze in dieser Richtung durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465].

Anionische grenzflächenaktive Verbindungen mit wenigstens zwei hydrophilen und wenigstens zwei hydrophoben Gruppen sind bisher nur auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Es bestand daher die Aufgabe, amphiphile Verbindungen herzustellen, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne großen Anfall von unerwünschten Nebenprodukten hergestellt werden können.

Die Aufgabe wird gelöst durch die zur Verfügungstellung von amphiphilen Verbindungen auf der Basis von Di-, Oligo- oder Polyenen.

Bei den erfindungsgemäßen amphiphilen Verbindungen handelt es sich um Verbindungen, die bei Einsatz von Alkylendienen typischerweise den allgemeinen Formeln I und II entsprechen. Für die Umsetzung mehrer Doppelbindungen ist das Ergebnis analog.

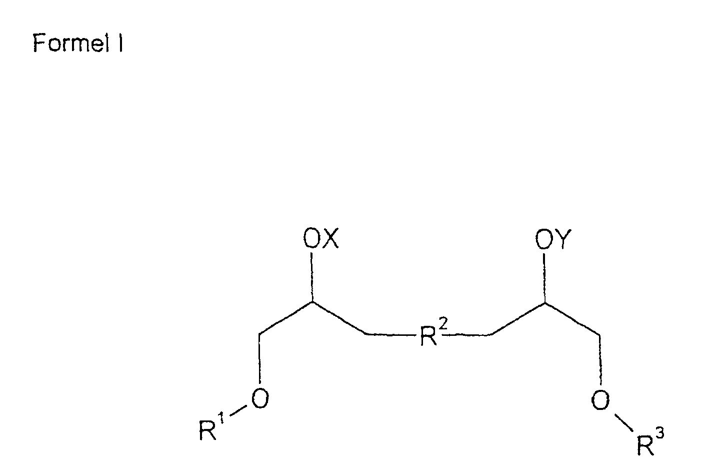

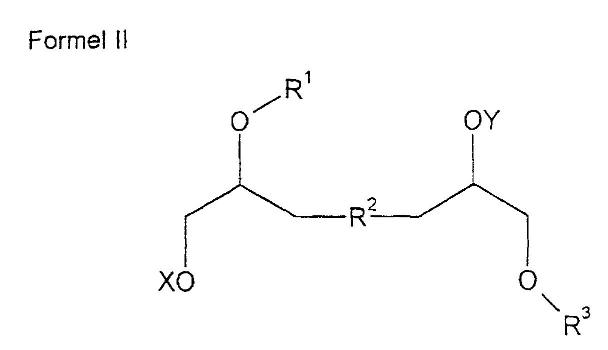

Gegenstand der Erfindung sind daher amphiphile Verbindungen der allgemeinen Formeln I und II oder bei Umsetzung mehrerer Doppelbindungen analogen Strukturen. 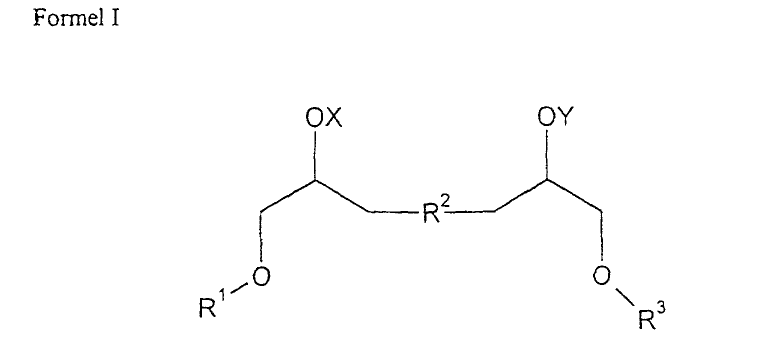 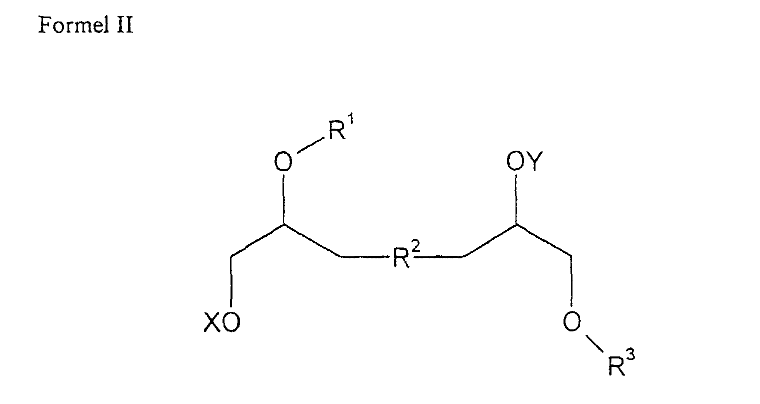 in der
R¹ und R³ unabhängig voneinander einen unverzweigten oder verzweigten, gesättigten Kohlenwasserstoffrest oder einen teil- oder perfluorierten Kohlenwasserstoffrest mit 1 bis 22, vorzugsweise 6 bis 18, Kohlenstoffatomen,
R² einen Spacer
und X und Y unabhängig voneinander Substituenten der Formel XV

   -(C₂H₄O)_{α}(C₃H₆O)_{β}H (XV)

   mit
   α = 0 bis 50, vorzugsweise α = 0 bis 15,
   β = 0 bis 60, vorzugsweise β = 0 bis 10,
   und α + β = 1 bis 100, vorzugsweise α + β = bis 20,
      oder der Formel XVI

      -(C₂H₄O)_{γ}(C₃H₆O)_{δ}-FR (XVI)
   mit
   γ = 0 bis 20, vorzugsweise γ = 0 bis 8,
   δ = 0 bis 20, vorzugsweise δ = 0 bis 12,
   und γ + δ = 0 bis 40, vorzugsweise γ + δ = 0 bis 20,
   wobei FR für einen funktionellen Rest -CH₂-COOM, -SO₃M, -P(O)(OM)₂, -C₃H₆-SO₃M oder -C(O)-C₂H₃(SO₃M)-CO₂M' mit M, M' = Alkali, Ammonium-, substituiertes Ammonium- oder ½ Erdalkaliion steht, bedeuten und wobei die Alkoxideinheiten statistisch oder blockweise eingebunden sind und die Reihenfolge beliebig ist.

Es seien als Substituenten R¹ und R³ im einzelnen die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Henicosyl, n-Docosyl und ihre verzweigtkettigen Isomeren und deren teil - oder perfluorierten Äquivalente genannt.

R² bedeutet einen Spacer, bestehend aus einer unverzweigten oder verzweigten Kette mit 2 bis 30 Kohlenstoffatomen, die 0 bis 10 Sauerstoff-, 0 bis 10 Stickstoff- und 0 bis 3 Schwefelatome enthält und die 0 bis 10 funktionelle Seitengruppen, wie z. B. Carbonyl-, Carboxyl-, Amino- und/oder Acylaminogruppen, aufweist.

Der Spacer R² bedeutet insbesondere
◆ als Grundkörper unverzweigte oder verzweigte Alkylenketten

   -CₐH₂ₐ- (III)

   mit a = 2 bis 18, vorzugsweise a = 2 bis 6;
◆ als Grundkörper Alicyclen gemäß der Formel IV

   -C_{f}H_{2f}-cyclo C₆H₁₀-C_{g}H_{2g}- (IV)

   mit f und g gleich unabhängig voneinander je 1 bis 6
   oder gemäß der Formel V

   -3(4),8(9)-di(methylen)-tricyclo[5.2.1.0^{2.6}]decan- (V);
◆ als Grundkörper gegebenenfalls substituierte Aromaten gemäß der Formel VI

   -CₕH₂ₕ-C₆R₄-(CᵢH₂ᵢ-C₆R₄)ⱼ₁-Cⱼ₂H₂ⱼ₂- (VI)

   oder gemäß der Formel VII

   -CₕH₂ₕ-C₁₀R₆-CⱼH₂ⱼ- (VII)

   mit h, j, j₁ und j₂ gleich unabhängig voneinander je 0 bis 8 und i = 0 bis 8 und mit R in den Formeln VI und VII gleich unabhängig voneinander jeweils H oder C₁- bis C₆-Alkyl;
◆ eine Kette mit funktionellen Seitengruppen, insbesondere Carbonyl-, Carboxyl-, Amino- und/oder Acylaminogruppen.

Weiterhin enthält der Spacer R² je 0 bis 10, vorzugsweise 1 bis 5, Sauerstoff- und/oder Stickstoffatome und/oder 0 bis 3 Schwefelatome.

R² hat damit weiterhin insbesondere die Bedeutung
◆ einer Verbindung gemäß der Formel VIII

   -CₖH₂ₖ-CₓR_{y}-Z-CₓR_{y}-CₗH₂ₗ- (VIII)

   mit k und l gleich unabhängig voneinander je 0 bis 8, R gleich unabhängig voneinander jeweils H oder C₁- bis C₆-Alkyl, x = 6 und y = 4 oder x = 10 und y = 6 oder x = 14 und y = 8, und

   Z = O, CO, NH, NR¹, N-C(O)R¹, SO₂

   oder gemäß der Formel IX

   -CH₂-CH(OCH₂CH(OX)-R¹)-CH₂- oder ein Isomer (IX)

   oder 2,2'-Methylen-bis-(1,3-dioxolan-5-methylen)-
   oder Acetale, insbesondere Diacetale aus Dialdehyden und Di-, Oligo- oder Polyolen, wobei R¹ einen Kohlenwasserstoffrest mit 1 bis 22 Kohlenstoffatomen bedeutet und X für einen Substituenten steht, der eine funktionelle Gruppe trägt und die weiter unten angegebene Bedeutung hat;
◆ einer Verbindung gemäß der Formel X

   -CₘH₂ₘ-(OCₙH₂ₙ)ₚ-C_{q}H_{2q}- (X)

   mit m = 1 bis 4, n = 2 bis 4, p = 1 bis 20, vorzugsweise p = 1 bis 4, und q = 1 bis 4, wobei auch gemischte Alkoxideinheiten auftreten können und dann die Reihenfolge der Alkoxideinheiten beliebig ist;
◆ einer Verbindung gemäß der Formel XI

   -CᵣH₂ᵣ(RNCₛH₂ₛ)ₜ-CᵤH₂ᵤ- (XI)

   oder gemäß der Formel XII

   -[CᵣH₂ᵣ[RN-C(O)-NR]ₜ-CᵤH₂ᵤ]_{w}- (XII)

   oder gemäß der Formel XIII

   -[CᵣH₂ᵣ[RNC(O)CᵥH₂ᵥC(O)NR]ₜ-CᵤH₂ᵤ]_{w}- (XIII)

   oder gemäß der Formel XIV

   -[CᵣH₂ᵣ[RNC(O)CₓR_{y}C(O)NR]ₜ-CᵤH₂ᵤ]_{w}- (XIV)

   mit r = 2 bis 4, s = 2 bis 4, t = 1 bis 20, vorzugsweise t = 1 bis 4, u = 2 bis 4, v = 0 bis 12, w = 1 bis 6, x = 6 und y = 4 oder x = 10 und y = 6 oder
   x = 14 und y = 8
   mit R gleich unabhängig voneinander H oder C₁- bis C₆-Alkyl,
   bedeutet.
   X und Y stehen unabhängig voneinander für Substituenten der Formel XV

   -(C₂H₄O)_{α}(C₃H₆O)_{β}H (XV)

   mit α = 0 bis 50, vorzugsweise α = 0 bis 15,
   β = 0 bis 60, vorzugsweise β = 0 bis 10,
   und
   α + β = 1 bis 100, vorzugsweise α + β = 1 bis 20, oder für Substituenten der Formel XVI

      -(C₂H₄O)_{γ}(C₃H₆O)_{δ}-FR (XVI)

      mit jeweils γ = 0 bis 20, vorzugsweise γ = 0 bis 8,
      δ = 0 bis 20, vorzugsweise δ = 0 bis 12,
      und γ + δ = 0 bis 40, vorzugsweise γ + δ = 0 bis 20,
   wobei FR für einen funktionellen Rest -CH₂-COOM, -SO₃M, -P(O)(OM)₂, -C₃H₆-SO₃M oder -C(O)-C₂H₃(SO₃M)-CO₂M'
   mit M, M' = Alkali, Ammonium-, substituiertes Ammonium oder ½ Erdalkali, steht
   und wobei die Alkoxideinheiten statistisch oder blockweise eingebunden sind und die Reihenfolge beliebig ist.

Der Alkoxylierungsgrad ist jeweils ein Mittelwert und kann jeden beliebigen, auch nicht ganzzahligen Wert in den angegebenen Grenzen einnehmen.

Ein weiterer Gegenstand der Erfindung ist ein Verfahren zur Herstellung der oben beschriebenen amphiphilen Verbindungen, wobei die Herstellung der amphiphilen Verbindungen in zwei Schritten erfolgt.

Es entstehen jeweils Mischungen dieser Verbindungen, weil die Öffnung der im erfindungsgemäßen Reaktionsverlauf entstehenden Epoxide nur mit einer Selektivität von 50 bis 90%, bevorzugt jedoch 70 bis 80%, von der weniger stark substituierten Seite erfolgt.

Im ersten Schritt erfolgt die Herstellung von Hydroxyethern (X, Y = H, Formel I bzw. II und entsprechenden analogen Strukturen) durch die Umsetzung von Di- oder Oligoenen, die cyclisch oder acyclisch sein können, mit organischen Hydroperoxiden, ROOH, als Oxidationsmittel und die Öffnung des entstandenen Oxiranringes durch ein- oder mehrwertige Alkohole in Gegenwart von homogenen oder heterogenen Molybdänverbindungen als erste Katalysatorkomponente und Bortrifluorid, als stabilisierter Komplex, oder eines Aluminiumoxids oder 1,8-Diazabicyclo-[5.4.0]-undec-7-en oder 1,4-Diazabicyclo-[2.2.2]-octan als zweite Katalysatorkomponente und der Verwendung von ein- oder mehrwertigen Alkoholen als Nucleophil sowie gleichzeitig als Lösungsmittel, wobei aber die Verwendung anderer Lösungsmittel nicht ausgeschlossen werden soll.

In einem zweiten Schritt werden die entstandenen Di- oder Oligoole erfindungsgemäß mit Hilfe von Alkoxilierungsmitteln zu nichtionischen Tensiden oder mit vorgeschalteter Alkoxylierung oder auf direktem Wege in anionische, amphiphile Verbindungen, überführt, indem man z. B. die vorgenannten Verbindungen mit Schwefeltrioxid/Inertgas, Oleum, Chlorsulfonsäure oder Amidosulfonsäure, mit Polyphosphorsäure, mit einer Halogenessigsäure oder mit Sauerstoff in Gegenwart eines TEMPO-Derivates (TEMPO-Derivate sind Derivate von 2,2',6,6'-Tetramethylpiperidinoxiden) oxidiert, mit einem Sulton, mit einem Taurin oder mit Maleinsäureanhydrid und Natriumhydrogensulfit umsetzt und jeweils anschließend mit wäßrigen Alkali- oder Erdalkalihydroxiden oder wäßrigem Ammoniak oder Alkanolaminen neutralisiert. Bei Bedarf werden die Produkte in wäßriger Lösung mit Wasserstoffperoxid (0,1 bis 2,0 % bezogen auf Feststoff) gebleicht.

Obwohl die einzelnen Komponenten der Reaktion bekannt sind, ist es überraschend, daß sie sich in einem "Eintopfverfahren" kombinieren lassen und bei der Reaktion selektiv der α-Hydroxyether gebildet wird. Die Katalysatorkomponenten beeinflussen sich gegenseitig nicht negativ, es ist sogar zu beobachten, daß BF₃ die Aktivität des Molybdänkatalysators für die Epoxidation erhöht.

Für das erfindungsgemäße Verfahren geeignete Katalysatorkomponenten für die Epoxidierung sind Molybdänverbindungen, die entweder im Reaktionsgemisch löslich sind, wie Molybdänylacetylacetonat, MoO₂(acac)₂ oder Molybdänhexacarbonyl, Mo(CO)₆, oder Molybdänoxid auf einem Katalysatorträger als heterogener Katalysator. Geeignete Katalysatorträger sind amorphe Alumosilikate oder Zeolithe mit hoher Lewis-Acidität. Der Molybdänkatalysator wird in einer Menge von 0,01 bis 5 mol%, bevorzugt 0,25 bis 2 mol% und besonders bevorzugt 0,5 bis 1,0 mol%, bezogen auf die zu oxidierende C=C-Doppelbindung, eingesetzt.

Als zweite erfindungsgemäße Katalysatorkomponente können Bortrifluorid oder Addukte, wie das Etherat oder das Methanolat eingesetzt werden. Ebenso eignen sich Aluminiumoxid, Al₂O₃, und die basischen Verbindungen 1,8-Diazabicyclo-[5.4.0]-undec-7-en oder 1,4-Diazabicyclo-[2.2.2]-octan. Diese Katalysatorkomponenten werden in einer Menge von 0,01 bis 5 mol%, bevorzugt 0,25 bis 2,0 mol% und besonders bevorzugt in einer Menge von 0,5 bis 1,0 mol%, bezogen auf die zu oxidierende C=C-Doppelbindung, eingesetzt.

Als olefinische Substrate können endständig und / oder innenständig zwei- oder mehrfach ungesättigte aliphatische, cyclische oder acyclische Kohlenwasserstoffe oder Fettsäuren sowie deren Ester eingesetzt werden. Zweckmäßigerweise sollte die Alkoholkomponente bei den Fettsäureestern gleich dem verwendeten Alkohol sein, damit die ebenfalls katalysierte Umesterung nicht zu ungewollten Produktgemischen führt.

Für das erfindungsgemäße Verfahren können ein- oder mehrwertige Alkohole mit primären, sekundären oder tertiären Hydroxylgruppen und beliebiger Kettenlänge eingesetzt werden. Sollen Verbindungen entsprechend der Formel (II) hergestellt werden, sollten nur primäre Hydroxylgruppen für die Veretherung eingesetzt werden, um unerwünschte Produktgemische zu vermeiden. Der Alkohol und das olefinische Substrat können weitere fünktionelle Gruppen, wie zum Beispiel Estergruppen, Carbonylkohlenstoffe, Amide, Ether, enthalten, solange diese in der Reaktion nicht wirkungsvoll als Nucleophile konkurrieren können.

Als Oxidationsmittel für das erfindungsgemäße Verfahren können die kommerziell erhältlichen Hydroperoxide, wie z.B tert.-Butylhydroperoxid oder Cumolhydroperoxid, verwendet werden. Das Oxidationsmittel wird im Verhältnis 1,0 bis 1,3 bezogen auf die zu oxidierenden Doppelbindungsäquivalente eingesetzt.

Die Reaktion nach dem erfindungsgemäßen Verfahren kann bei Temperaturen beginnend beim Schmelzpunkt des Reaktionsgemisches und endend beim Siedepunkt des Reaktionsgemisches durchgeführt werden. Weiterhin wird die Reaktion bevorzugt in einer Schutzgasatmosphäre und mit vom Wasser befreiten Reagenzien durchgeführt. Zur Reaktion werden die Katalysatorkomponenten und Alkohol sowie Olefin gemischt und auf die Reaktionstemperatur erwärmt. Das Hydroperoxid wird dann langsam zudosiert. Nach Beendigung der Reaktion können die Katalysatorkomponenten im einfachsten Fall, beide heterogen, abfiltriert und weiter verwendet werden oder sie müssen mit Wasser aus dem Produkt entfernt werden. Die entstandenen α-Hydroxyether können i.a. durch Destillation aufgereinigt werden, was aber häufig nicht notwendig ist.

Die erfindungsgemäßen amphiphilen Verbindungen zeichnen sich meist durch extrem niedrige kritische Micellbildungskonzentrationen (CMC) und sehr niedrige Oberflächen- und Grenzflächenspannungen (z. B. gegen Paraffin) aus, was auf ihre besondere Struktur - wenigstens zwei hydrophile Gruppen und wenigstens zwei hydrophobe Gruppen - zurückgeführt werden muß. Darüber hinaus weisen die meisten von ihnen ein recht hohes hydrophiles Suspendiervermögen auf und sind ausgesprochen hautmild. Einige dieser Verbindungen sind extrem schnelle Netzmittel.

Die amphiphilen Verbindungen gemäß dieser Erfindung eignen sich insbesondere als Emulgatoren, Demulgatoren, Detergenzien, Dispergatoren und Hydrotropica oder Cryptanden (im Falle der cyclischen Verbindungen) in Industrie und Haushalt.

Ein weiterer Gegenstand der Erfindung ist daher die Verwendung der amphiphilen Verbindungen auf den Gebieten Erzgewinnung, Metallbearbeitung, Oberflächenveredelung, Kunststoffherstellung oder Kunststoffverarbeitung, Kosmetik, Medizin, Nahrungsmittelverarbeitung und -zubereitung, als Hilfsmittel bei der Ausbringung von Pflanzenbehandlungsmitteln, als Hilfsmittel bei medizinischen Anwendungen, als Textilhilfsmittel, als Hilfsmittel für das Reinigen und Waschen von Textilien, als Hilfsmittel für das Reinigen von harten Oberflächen und als Hilfsmittel für das Reinigen und Waschen von Haut und Haar.

Hierbei können sie mit allen gängigen anionischen, nichtionischen, kationischen und ampholytischen grenzflächenaktiven Substanzen kombiniert werden. Als Beispiele für nichtionische grenzflächenaktive Substanzen, die für eine Kombination eingesetzt werden können, seien Fettsäureglyceride, Fettsäurepolyglyceride, Fettsäureester, Ethoxylate höherer Alkohole, Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate, Polyoxyethylenlanolinderivate, Polyoxyethylenfettsäureamide, Polyoxyethylenalkylamine, Alkanolamine, Alkylaminoxide, Derivate von Eiweißhydrolysaten, Hydroxymischether, Alkylpolyglycoside und Alkylglucamide genannt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, Sulfonate höherer Fettsäureester, höhere Alkoholsulfate, Alkoholethersulfate, Hydroxymischethersulfate, Salze von Phosphatestern, Tauride, Isethionate, lineare Alkylbenzolsulfonate, Alkylarylsulfonate, Sulfate der Polyoxyethylenfettsäureamide und Salze von Acylaminosäuren genannt.

Als Beispiele für kationische gängige grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate genannt.

Als Beispiele für ampholytische grenzflächenaktive Substanzen, die für Kombinationen eingesetzt werden können, seien Aminosäuren, Betaine, Sulfobetaine, Imidazolinderivate, Sojaöllipide und Lecithin genannt.

Darüber hinaus können die erfindungsgemäßen amphiphilen Verbindungen auch für sich miteinander kombiniert werden.

Den erfindungsgemäßen amphiphilen Verbindungen können ebenfalls gängige Additive zugesetzt werden. Solche Additive werden speziell für eine Formulierung ausgewählt und umfassen üblicherweise anorganische Salze, wie Natriumchlorid und -sulfat, sowie Builder, Hydrotropica wie Cumolsulfonat, UV-Absorber, Weichmacher, Chelatbildner, Viskositätsmodifizierer, Riechstoffe usw.

Folgendes Beispiel soll die Erfindung erläutern, sie jedoch nicht darauf einschränken.

### Beispiel:

### Epoxidierung und Ringöffnung:

### Herstellung von Dibutoxyoctandiol

0,05 mol 1,7-Octadien (5,9 g) und 1 mmol MoO₂(acac)₂ (326 mg) werden in 60 ml abs. 1-Butanol gelöst, eine Spatelspitze trockenes Molsieb (4Å) zugegeben und auf 90 °C erwärmt. Anschließend werden 0,15 ml BF₃-Etherat ( ≈ 1 mmol) und tropfenweise innerhalb von 30 min 0,12 mol abs. tert.-Butylhydroperoxid (5M in Decan, 24 ml) zugegeben. Man läßt noch 16 h nachreagieren, kühlt ab, säuert mit verdünnter Salzsäure an, nimmt in Ether auf und wäscht die organische Phase mit Wasser. Das verbleibende Rohprodukt wird über Na₂SO₄ getrocknet und einer gaschromatographsichen Analyse unterworfen. Nach Zusatz eines GC-Standards (Heptansäureethylester) und nach Überführung der freien Carbonsäuren mit CH₂N₂ in die entsprechenden Methylester werden unter Berücksichtigung vorher mittels reiner Substanzen ermittelter Korrekturfaktoren die gebildeten Mengen der Produkte quantitativ bestimmt.
Ausbeute an Dibutoxyoctandiol: 49 % der Theorie bez. auf 1,7-Octadien (3 Isomere) (die Nebenprodukte Epoxybutoxyoctanol, Diepoxyoctan, Butoxyoctenol und Butoxyoctantriol fallen nur in Anteilen von < 5 % an).

### Sulfatierung:

Zu einem Gemisch aus 61,3 g Chlorsulfonsäure und 31,8 g Essigsaure werden 49,6 g des oben erhaltenen Diols in 200 ml Dichlormethan so zugetropft, daß die Temperatur 5 °C nicht übersteigt. Anschließend wird bei Raumtemperatur 3 Stunden gerührt (laut Kontrolle per Dünnschichtchromatographie ist das Diolgemisch vollständig umgesetzt). Es wird mit 2-normaler Natriumcarbonatlösung neutralisiert und mit gesättigter Natriumhydrogencarbonatlösung verdünnt. Mit n-Butanol wird das Produkt extrahiert und anschließend der Alkohol entfernt. Die Reinheitskontrolle erfolgt per Dünnschichtchromatographie und NMR. Ausbeute: 70,3 g (80 % d. Th.), Reinheit: 90 % (Isomerengemisch).

## Patentansprüche

1. Amphiphile Verbindung der allgemeinen Formel I oder II 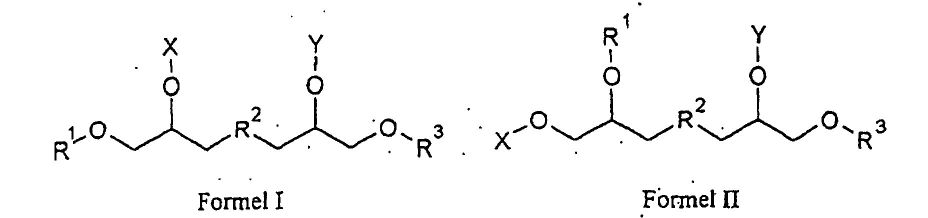 in der
R¹ und R³ unabhängig voneinander einen unverzweigten oder verzweigten, gesättigten Kohlenwasserstoffrest mit 1 bis 22 Kohlenstoffatomen oder einen teil- oder perfluorierten Kohlenwasserstoffrest mit 1 bis 22 Kohlenstoffatomen,
R² einen Spacer, der eine unverzweigte oder verzweigte Kette mit 2 bis 30 Kohlenstoffatomen, die 0 bis 10 Sauerstoff-, 0 bis 10 Stickstoff- und 0 bis 3 Schwefelatome enthält und die 0 bis 10 funktionelle Seitengruppen aufweist und
X und Y unabhängig voneinander einen Substituenten
der Formel XV
-(C₂H₄O)_{α}(C₃H₆O)_{β}H (XV)
mit
α = 0 bis 50,
β = 0 bis 60 und
α + β = 1 bis 100,
oder der Formel XVI
-(C₂H₄O)_{γ}(C₃H₆O)_{δ}-FR (XVI)
mit
γ = 0 bis 20
δ = 0 bis 20 und
und γ + δ = 0 bis 40
wobei FR für einen funktionellen Rest -CH₂-COOM, -SO₃M, -P(O)(OM)₂, -C₃H₆-SO₃M oder -C(O)-C₂H₃(SO₃M)-CO₂M mit M, M = Alkali-, Ammonium-, Alkanolammonium- oder ½ Erdalkaliion steht,
bedeutet und wobei die Alkoxideinheiten statistisch oder blockweise eingebunden sind und die Reihenfolge beliebig ist.

2. Amphiphile Verbindungen nach Anspruch 1, **dadurch gekennzeichnet, daß** in dem Spacer
R² je 1 bis 5 Sauerstoff- und/oder Stickstoffatome und/oder 0 bis 3 Schwefelatome enthalten sind.

3. Amphiphile Verbindungen nach einem der Ansprüche 1 bis 2, **dadurch gekennzeichnet, daß** die Reste
R¹ und R³ unabhängig voneinander 6 bis 18 Kohlenstoffatome enthalten

4. Amphilphile Verbindungen nach einem der Ansprüche 1 bis 3, **dadurch gekennzeichnet, daß**
R² einen Spacer bedeutet, der als Grundkörper eine unverzweigte oder verzweigte Alkylenkette der Formel III
-CₐH₂ₐ- (III)
mit a = 2 bis 18
enthält.

5. Amphiphile Verbindungen nach einem der Ansprüche 1 bis 3, **dadurch gekennzeichnet, daß**
R² einen Spacer bedeutet, der als Grundkörper aus Alicyclen gemäß der Formel IV
-C_{f}H_{2f}-cyclo C₆H₁₀-C_{g}H_{2g}- (IV)
mit f und g gleich unabhängig voneinander je 1 bis 6
oder gemäß der Formel V
-3(4),8(9)-Di(methylen)-tricyclo[5.2.1.0^{2.6}]decan- (V)
besteht.

6. Amphiphile Verbindungen nach einem der Ansprüche 1 bis 3, **dadurch gekennzeichnet, daß**
R² einen Spacer bedeutet, der als Grundkörper aus unsubstituierten oder substituierten Aromaten gemäß der Formel VI
-CₕH₂ₕ-C₆R₄-(CᵢH₂ᵢ-C₆R₄)_{J¹}-C_{J²}H_{2J²}- (VI)
oder gemäß der Formel VII
-CₕH₂ₕ-C₁₀R₆-CⱼH₂ⱼ- (VII)
mit h, j, j₁ und j₂ gleich unabhängig voneinander je 0 bis 8 und i = 0 bis 8 und R gleich unabhängig voneinander jeweils H oder C₁-bis C₆-Alkyl besteht.

7. Amphiphile Verbindungen nach einem der Ansprüche 1 bis 6, **dadurch gekennzeichnet, daß** der Spacer
R² als funktionelle Seitengruppen, Carbonyl-, Carboxyl-, Amino- und/oder Acylaminogruppen, trägt.

8. Amphiphile Verbindungen nach Anspruch 1, **dadurch gekennzeichnet, daß**
R² einen Spacer gemäß der Formel (VIII)
-CₖH₂ₖ-CₓR_{y}-Z-CₓR_{y}-CₗH₂ₗ- (VIII)
mit
k und l gleich unabhängig voneinander je 0 bis 8,
R gleich unabhängig voneinander jeweils H oder C₁- bis C₆-Alkyl,
x = 6 und y = 4 oder x = 10 und y = 6 oder x = 14 und y = 8,
und Z = O, CO, NH, NR¹, N-C(O)R¹ oder SO₂
oder gemäß der Formel IX
-CH₂-CH(OCH₂CH(OX)-R¹)-CH₂- oder ein Isomer (IX),
oder 2,2 -Methylen-bis-(1,3-dioxolan-5-methylen)-
oder Diacetale aus Dialdehyden und Di-, Oligo- oder Polyolen bedeutet,
wobei R¹ einen Kohlenwasserstoffrest mit 1 bis 22 Kohlenstoffatomen bedeutet und X für einen Substituenten steht, der eine funktionelle Gruppe trägt und die Bedeutung gemäß der Formel XVI hat.

9. Amphiphile Verbindung nach Anspruch 1, **dadurch gekennzeichnet, daß**
R² einen Spacer gemäß der Formel X
-CₘH₂ₘ-(CₙH₂ₙO)ₚ-C_{q}H_{2q}- (X)
mit m = 1 bis 4, n = 2 bis 4, p = 1 bis 20 and q = 1 bis 4, wobei auch gemischte Alkoxideinheiten auftreten können und dann die Reihenfolge der Alkoxideinheiten beliebig ist, bedeutet.

10. Amphiphile Verbindungen nach Anspruch 1, **dadurch gekennzeichnet, daß**
R² einen Spacer gemäß der Formel XI
-CᵣH₂ᵣ(RNCₛH₂ₛ)ₜ₋CᵤH₂ᵤ- (XI)
oder gemäß der Formel XII
-[CᵣH₂ᵣ[RNC(O)-NR]ₜ-CᵤH₂ᵤ]_{w}- (XII)
oder gemäß der Formel XIII
-[CᵣH₂ᵣ[RNC(O)CᵥH₂ᵥC(O)NR]ₜ-CᵤH₂ᵤ]_{w₋} (XIII)
oder gemäß der Formel XIV
-[CᵣH₂ᵣ[RNC(O)CₓR_{y}C(O)NR]ₜ-CᵤH₂ᵤ]_{w}- (XIV)
mit
r = 2 bis 4, s = 2 bis 4, t = 1 bis 20, u = 2 bis 4,
v = 0 bis 12, w = 1 bis 6, x = 6 und y = 4 oder
x = 10 und y = 6 oder
x = 14 und y = 8
mit R gleich unabhängig voneinander H oder C₁-bis C₆-Alkyl, bedeutet.

11. Verfahren zur Herstellung von amphiphilen Verbindungen, umfassend die Schritte
(1) Herstellen von Hydroxyethern des Typs 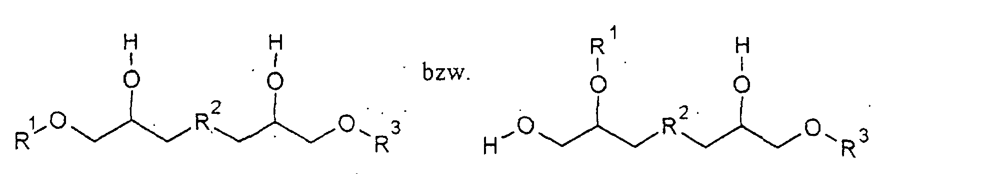 durch Umsetzen
- eines olefinischen Substrats,
wobei das olefinische Substrat ein mehrfach ungesättigter Kohlenwasserstoff oder eine mehrfach ungesättigte Fettsäure / Fettsäureester ist und das olefinische Substrat weiterhin Estergruppen, Carbonylkohlenstoffe, Amid- und/oder Ether- Gruppen enthalten kann,
- mit organischen Hydroperoxiden des Typs ROOH
und Öffnen des entstandenen Oxiranringes durch
- ein oder mehrwertige Alkohole,
die ggf. Estergruppen, Carbonylkohlenstoffe, Amid-, Ether-Gruppen enthalten können und/oder teil- oder perfluoriert sein können,
in Gegenwart einer
- Molybdänverbindung als erster Katalysatorkomponente und
- Bortrifluorid, einem Aluminiumoxid, 1,8-Diazobicyclo-[5.4.0]-undec-7-en
oder 1,4-Diazobicyclo-[2.2.2]-octan als zweiter Katalysatorkomponente
und
(2.1) Alkoxylieren der Hydroxyether mit einem
- Alkoxylierungsmittel zu einer amphiphilen nicht-ionischen Verbindung
oder
(2.2) Umsetzen der Hydroxyether zu einer amphiphilen ionischen Verbindung (a), ggf. mit vorgeschalteter Alkoxylierung (b).

12. Verfahren zur Herstellung von amphiphilen Verbindungen gemäß Anspruch 11, **dadurch gekennzeichnet, daß** die Herstellung der amphiphilen ionischen Verbindung (2.2) durch
Umsetzen der Hydroxyether gemäß einem der folgenden Schritte erfolgt:
(a) Umsetzen der Hydroxyether mit
- Schwefeltrioxid/Inertgas; Oleum; Chlorsulfonsäure; Amidosulfonsäure; Phosphorsäure; Sauerstoff in Gegenwart eines Derivates der 2,2',6,6'-Tetramethylpiperidinoxides; Halogenessigsäure; einem Sulton; einem Taurin und/oder Maleinsäureanhydrid gefolgt von Natriumhydrogensulfid
- und ggf. Neutralisieren
oder
(b) Alkoxylieren gefolgt von Schritt (a).

13. Verfahren gemäß einem der Ansprüche 11 oder 12 zur Herstellung einer amphiphilen Verbindungen gemäß einem der Ansprüche 1 bis 10.

14. Verfahren gemäß einem der Ansprüche 11 bis 13, **dadurch gekennzeichnet, daß** man die Reaktion als Eintopf-Reaktion durchführt.

15. Verfahren nach einem der Ansprüche 11 bis 14, **dadurch gekennzeichnet, daß** man einen ein- oder mehrwertigen Alkohol als Lösungsmittel einsetzt.

16. Verfahren nach einem der Ansprüche 11 bis 14, **dadurch gekennzeichnet, daß** man unter Wasserausschluß arbeitet.

17. Verfahren nach einem der Ansprüche 11 bis 14, **dadurch gekennzeichnet, daß** man lösliche Molybdänverbindungen verwendet.

18. Verfahren nach einem der Ansprüche 11 bis 14, **dadurch gekennzeichnet, daß** man MoO₂(acac)₂ oder Mo(CO)₆ verwendet.

19. Verfahren nach einem der Ansprüche 11 bis 14, **dadurch gekennzeichnet, daß** man Molybdanoxid auf einem Katalysatorträger einsetzt.

20. Verfahren nach Anspruch 19, **dadurch gekennzeichnet, daß** man als Katalysatorträger amorphe Alumosilikate oder Zeolithe einsetzt.

21. Verwendung der amphiphilen Verbindungen nach einem der Ansprüche 1 bis 10 bei der Ausbringung von Pflanzenbehandlungsmitteln, bei medizinischen Anwendungen oder bei der Kunststoffverarbeitung.

## Claims

1. Amphiphilic compounds of the general formula I or II 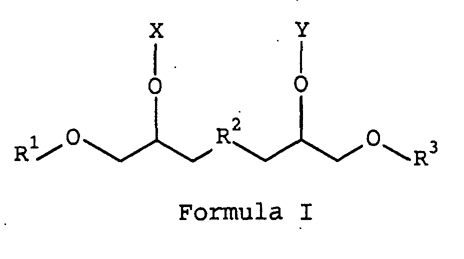 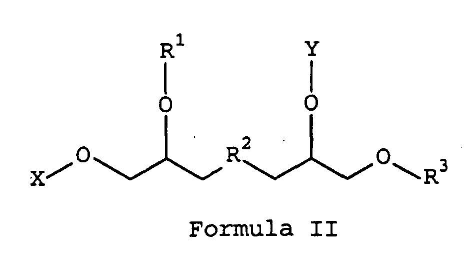 where
**R**^{**1**} and **R**^{**3**}, independently of one another, represent an unbranched or branched, saturated hydrocarbon radical with 1 to 22 carbon atoms or a partially fluorinated or perfluorinated hydrocarbon radical with 1 to 22 carbon atoms,
**R**^{**2**} is a spacer comprising an unbranched or branched chain with 2 to 30 carbon atoms, which has from 0 to 10 oxygen atoms, 0 to 10 nitrogen atoms and 0 to 3 sulfur atoms, and 0 to 10 functional side groups, and
**X** and **Y,** independently of one another, are a substituent of the formula XV
- (C₂H₄O)_{α}(C₃H₆O)_{β}H **(XV)**
where
α = 0 to 50,
β = 0 to 60, and
α + β = 1 to 100,
or of the formula XVI
-(C₂H₄O)_{γ}(C₃H₆O)_{δ}-FR **(XVI)**
where
γ = 0 to 20,
δ = 0 to 20, and
γ + δ = 0 to 40,
and FR represents a functional radical -CH₂-COOM, -SO₃M,
-P(O)(OM)₂, -C₃H₆-SO₃M, or
-C(O)-C₂H₃(SO₃M)-CO₂M'
with M, M' equal to alkali-, ammonium-, alkanol ammonium-, or ½ alkaline earth metal ion,
and where the alkoxide units are incorporated randomly or blockwise and the sequence is arbitrary.

2. Amphiphilic compounds as claimed in claim 1,
**characterized in that**
the spacer **R**^{**2**} comprises in each case 1 to 5 oxygen and/or nitrogen atoms and/or 0 to 3 sulfur atoms.

3. Amphiphilic compounds as claimed in any one of claims 1 to 2,
**characterized in that**
the radicals **R**^{**1**} and **R**^{**3**}, independently of one another, comprise 6 to 18 carbon atoms.

4. Amphiphilic compounds as claimed in any one of claims 1 to 3,
**characterized in that**
**R**^{**2**} is a spacer comprising as basic body an unbranched or branched alkylene chain of the formula III
-CₐH₂ₐ- **(III)**
where **a** = 2 to 18.

5. Amphiphilic compounds as claimed in any one of claims 1 to 3,
**characterized in that**
**R**^{**2**} is a spacer consisting as basic body of alicyclic compounds of the formula IV
-C_{f}H_{2f}-cycloC₆H₁₀-C_{g}H_{2g}- **(IV)**
where each **f** and **g,** independently of one another, is equal to from 1 to 6
or of the formula V
-3(4),8(9)-di(methylene)-tricyclo[5.2.1.0^{2.6}] decane- **(V).**

6. Amphiphilic compounds as claimed in any one of claims 1 to 3,
**characterized in that**
**R**^{**2**} is a spacer consisting as basic body of unsubstituted or substituted aromatics of the formula VI
-CₕH₂ₕ-C₆R₄-(CᵢH₂ᵢ-C₆R₄)ⱼ₁-Cⱼ₂H₂ⱼ₂- **(VI)**
or of the formula VII
-CₕH₂ₕ-C₁₀R₆-CⱼH₂ⱼ- **(VII)**
where each **h, j, j**_{**1**}**,** and **j**_{**2**}**,** independently of one another, is equal to from 0 to 8, and **i** is equal to from 0 to 8, and each **R,** independently of one another, is equal to H or C₁- to C₆-alkyl.

7. Amphiphilic compounds as claimed in any one of claims 1 to 6,
**characterized in that**
the spacer **R**^{**2**} bears carbonyl, carboxyl, amino and/or acylamino groups as functional side groups.

8. Amphiphilic compounds as claimed in claim 1,
**characterized in that**
**R**^{**2**} represents a spacer of the formula (VIII)
-CₖH₂ₖ-CₓR_{y}-Z-CₓR_{y}-CₗH₂ₗ- **(VIII)**
wherein each **k** and **l**, independently of one another, is equal to from 0 to 8,
each **R,** independently of one another, is equal to H or C₁- to C₆-alkyl,
**x** = 6 and **y** = 4, or
**x** = 10 and **y** = 6, or
**x** =14 and **y** = 8, and
**Z** = O, CO, NH, NR¹, N-C(O)R¹, SO₂
or of the formula IX
-CH₂-CH(OCH₂CH(OX)-R¹)-CH₂- or an isomer **(IX)**
or 2,2'-methylene-bis-(1,3-dioxolane-5-methylene)-, or diacetals of dialdehydes and di-, oligo-, or polyols,
where **R**^{**1**} is a hydrocarbon radical with 1 to 22 carbon atoms, and **X** is a substituent bearing a functional group and having the meaning of formula XVI.

9. Amphiphilic compounds as claimed in claim 1, **characterized in that**
**R**^{**2**} represents a spacer of the formula X
-CₘH₂ₘ-(CₙH₂ₙO)ₚ-C_{q}H_{2q}- (**X**)
where **m** = 1 to 4, **n** *=* 2 to 4, **p** = 1 to 20, and **q** = 1 to 4,
and wherein mixed alkoxide units may also be present and if so, the sequence of said alkoxide units is arbitrary.

10. Amphiphilic compounds as claimed in claim 1, **characterized in that**
**R**^{**2**} represents a spacer of the formula XI
-CᵣH₂ᵣ(RNCₛH₂ₛ)ₜ-CᵤH₂ᵤ- **(XI)**
or of the formula XII
- [CᵣH₂ᵣ[RN-C(O)-NR]ₜ-CᵤH₂ᵤ]_{w}- **(XII)**
or of the formula XIII
- [CᵣH₂ᵣ[RNC(O)CᵥH₂ᵥC(O)NR]ₜ-CᵤH₂ᵤ]_{w}- **(XIII)**
or of the formula XIV
-[CᵣH₂ᵣ[RNC(O)CₓR_{y}C(O)NR]ₜ-CᵤH₂ᵤ]_{w}- **(XIV)**
where **r** = 2 to 4, **s** = 2 to 4, **t** = 1 to 20,
**u** = 2 to 4, **v** = 0 to 12, **w** = 1 to 6,
**x** = 6 and **y** = 4, or
**x** = 10 and **y** = 6, or
**x** = 14 and **y** = 8,
and **R,** independently of one another, is equal to H or C₁- to C₆-alkyl.

11. Process for preparing amphiphilic compounds which process comprises the steps
(1) Preparation of hydroxyethers of the type 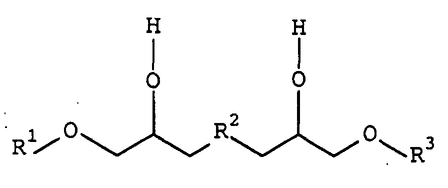 or 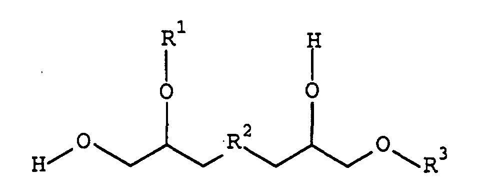 by reacting
- an olefinic substrate,
which is a polyunsaturated hydrocarbon or a polyunsaturated fatty acid/fatty acid ester, and which may furthermore comprise ester groups, carbonyl carbons, amide and/or ether groups,
- with organic hydroperoxides of the type ROOH, and opening the resultant oxirane ring by mono- or polyhydric alcohols, which may optionally comprise ester groups, carbonyl carbons, amide groups, ether groups, and/or may be partially fluorinated or perfluorinated
in the presence of
- a molybdenum compound as the first catalyst component and
- boron trifluoride, an alumina, 1,8-diazobi cyclo-[5.4.0]-undec-7-ene or 1,4-diazobicyclo-[2.2.2]-octane as the second catalyst component
and
(2.1) alkoxylation of the hydroxyethers with an alkoxylating agent to obtain an amphiphilic nonionic compound
or
(2.2) reaction of the hydroxyethers to form an amphiphilic ionic compound (a), optionally preceded by alkoxylation (b).

12. Process for preparing amphiphilic compounds as claimed in claim 11,
**characterized in that**
the preparation of the amphiphilic ionic compound (2.2) is carried out by
reaction of the hydroxyethers in accordance with one of the following steps:
(a) Reaction of the hydroxyethers with sulfur trioxide/inert gas; oleum; chlorosulfonic acid; sulfamic acid; phosphoric acid; oxygen in the presence of a derivative of 2,2',6,6'-tetramethylpiperidine oxide; haloacetic acid; a sultone; a taurine; and/or maleic anhydride followed by sodium bisulfide; and optionally neutralisation
or
(b) alkoxylation followed by step (a).

13. Process as claimed in claim 11 or 12 for preparing an amphiphilic compound as claimed in any one of claims 1 to 10.

14. Process as claimed in any one of claims 11 to 13,
**characterized in that**
the reaction is carried out as a one-pot reaction.

15. Process as claimed in any one of claims 11 to 14,
**characterized in that**
a monohydric or polyhydric alcohol is employed as a solvent.

16. Process as claimed in any one of claims 11 to 14,
**characterized in that**
water is excluded.

17. Process as claimed in any one of claims 11 to 14,
**characterized in that**
soluble molybdenum compounds are used.

18. Process as claimed in any one of claims 11 to 14, **characterized in that**
MoO₂(acac)₂ or Mo(CO)₆ is used.

19. Process as claimed in any one of claims 11 to 14,
**characterized in that**
molybdenum oxide on a catalyst carrier is employed.

20. Process as claimed in claim 19,
**characterized in that**
amorphous aluminosilicates or zeolites are employed as catalyst carriers.

21. Use of the amphiphilic compounds as claimed in any one of claims 1 to 10 in the application of crop protectants, medical applications, or plastics processing.

## Revendications

1. Composé amphiphile de formule générale I ou II 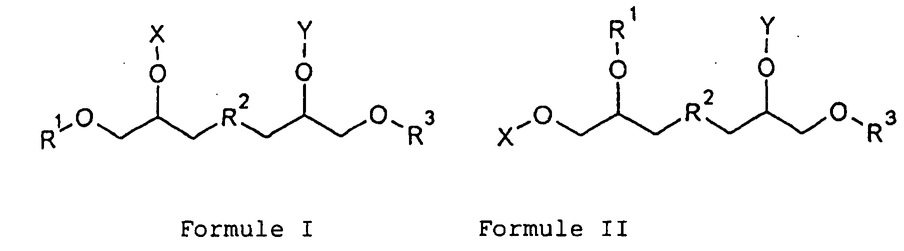 dans laquelle
R¹ et R³ représentent, indépendamment l'un de l'autre, un radical hydrocarboné saturé, linéaire ou ramifié, comprenant 1 à 22 atomes de carbone ou un radical hydrocarboné partiellement fluoré ou perfluoré comprenant 1 à 22 atomes de carbone,
R² représente un espaceur qui présente une chaîne linéaire ou ramifiée comprenant 2 à 30 atomes de carbone, contenant 0 à 10 atomes d'oxygène, 0 à 10 atomes d'azote et 0 à 3 atomes de soufre et qui présente 0 à 10 groupements latéraux fonctionnels et
X et Y représentent, indépendamment l'un de l'autre un substituant de formule XV
- (C₂H₄O)_{α}(C₃H₆O₆)_{β}H (XV)
avec
α = 0 à 50
β = 0 à 60 et
α + β = 1 à 100
ou de formule XVI
-(C₂H₄O)_{γ}(C₃H₆O)_{δ}FR (XVI)
avec
γ = 0 à 20
δ = 0 à 20 et
γ + δ = 0 à 40
FR représentant un radical fonctionnel -CH₂-COOM, -SO₃M, -P(O)(OM)₂, -C₃H₆-SO₃M ou -C(O)-C₂H₃(SO₃M)-CO₂M' avec M, M' = un ion alcalin, ammonium, alcanolammonium ou 1/2-alcalino-terreux,
et les unités alcoxyde étant liées statistiquement ou par blocs et l'ordre étant quelconque.

2. Composés amphiphiles selon la revendication 1, **caractérisés en ce que** dans l'espaceur R² sont contenus à chaque fois 1 à 5 atomes d'oxygène et/ou d'azote et/ou 0 à 3 atomes de soufre.

3. Composés amphiphiles selon l'une quelconque des revendications 1 à 2, **caractérisés en ce que** les radicaux R¹ et R³ contiennent indépendamment l'un de l'autre 6 à 18 atomes de carbone.

4. Composés amphiphiles selon l'une quelconque des revendications 1 à 3, **caractérisés en ce que** R² signifie un espaceur, qui contient comme corps de base une chaine alkylène linéaire ou ramifiée de formule III
-CₐH₂ₐ- (III)
avec a = 2 à 18.

5. Composés amphiphiles selon l'une quelconque des revendications 1 à 3, **caractérisés en ce que** R² signifie un espaceur qui est constitué comme corps de base d'alicycles selon la formule IV
-C_{f}H_{2f}-cyclo C₆H₁₀-C_{g}H_{2g}- (IV)
avec f et g représentant chacun indépendamment de l'autre 1 à 6,
ou selon la formule V
-3(4),8(9)-di(méthylène)-tricyclo[5.2.1.0^{2.6}]décane- (V)

6. Composés amphiphiles selon l'une quelconque des revendications 1 à 3, **caractérisés en ce que** R² signifie un espaceur qui est constitué comme corps de base d'aromates non substitués ou substitués selon la formule VI
-CₕH₂ₕ-C₆R₄-(CᵢH₂ᵢ-C₆R₄)ⱼ₁-Cⱼ₂H₂ⱼ₂- (VI)
ou selon la formule VII
-CₕH₂ₕ-C₁₀R₆-CⱼH₂ⱼ- (VII)
avec h, j, j₁ et j₂ représentant chacun indépendamment l'un de l'autre 0 à 8 et i = 0 à 8 et R représentant indépendamment l'un de l'autre à chaque fois H ou alkyle en C₁ à C₆.

7. Composés amphiphiles selon l'une quelconque des revendications 1 à 6, **caractérisés en ce que** l'espaceur R² porte comme groupements latéraux fonctionnels des groupements carbonyle, carboxyle, amino et/ou acylamino.

8. Composés amphiphiles selon la revendication 1, **caractérisés en ce que** R² signifie un espaceur selon la formule (VIII)
-CₖH₂ₖ-CₓR_{y}-Z-CₓR_{y}-CₗH₂ₗ- (VIII)
avec
k et l représentant chacun indépendamment l'un de l'autre 0 à 8, R représentant indépendamment l'un de l'autre à chaque fois H ou alkyle en C₁ à C₆
x = 6 et y = 4 ou
x = 10 et y = 6 ou
x = 14 et y = 8 et
Z = O, CO, NH, NR¹, N-C(O)R¹ ou SO₂,
ou selon la formule IX
-CH₂-CH(OCH₂CH(OX)-R¹)-CH₂- ou un isomère (IX)
ou 2,2-méthylène-bis-(1,3-dioxolane-5-méthylène)- ou des diacétals de dialdéhydes et de diols, d'oligools ou de polyols,
R¹ signifiant un radical hydrocarboné comprenant 1 à 22 atomes de carbone et X représentant un substituant qui porte un groupement fonctionnel et qui présente la signification selon la formule XVI.

9. Composé amphiphile selon la revendication 1, **caractérisé en ce que** R² signifie un espaceur selon la formule X
-CₘH₂ₘ-(OCₙH₂ₙ)ₚ-C_{q}H_{2q}- (X)
avec m = 1 à 4, n = 2 à 4, p = 1 à 20, et q = 1 à 4,
des unités mixtes d'alcoxydes pouvant également se présenter et l'ordre des unités alcoxydes étant alors quelconque.

10. Composés amphiphiles selon la revendication 1, **caractérisés en ce que** R² signifie un espaceur selon la formule XI
-CᵣH₂ᵣ(RNCₛH₂ₛ)ₜ-CᵤH₂ᵤ- (XI)
ou selon la formule XII
-[CᵣH₂ᵣ[RNC(O)-NR]ₜ-CᵤH₂ᵤ]_{w}- (XII)
ou selon la formule XIII
-[CᵣH₂ᵣ[RNC(O)CᵥH₂ᵥC(O)NR]ₜ-CᵤH₂ᵤ]_{w}- (XIII)
ou selon la formule XIV
-[CᵣH₂ᵣ[RNC(O)CₓR_{y}C(O)NR]ₜ-CᵤH₂ᵤ]_{w}- (XIV)
avec
r = 2 à 4, s = 2 à 4, t = 1 à 20, u = 2 à 4, v = 0 à 12, w = 1 à 6, x = 6 et y = 4 ou
x = 10 et y = 6 ou
x = 14 et y =8
avec R représentant indépendamment l'un de l'autre H ou alkyle en C₁ à C₆.

11. Procédé pour la préparation de composés amphiphiles comprenant les étapes
(1) préparation d'hydroxyéthers du type ou selon le cas 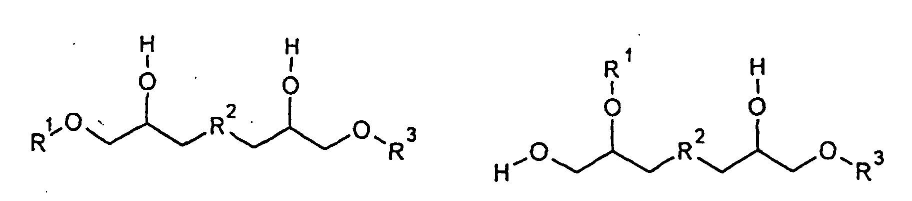 par transformation d'un substrat oléfinique, le substrat oléfinique étant un hydrocarbure polyinsaturé ou un acide gras/ester d'acide gras polyinsaturé et le substrat oléfinique pouvant en outre contenir des groupements ester, des carbones carbonyle, des groupements amide et/ou éther,
avec des hydroperoxydes organiques du type ROOH
et ouverture du cycle oxiranne formé par
des alcools monovalents ou polyvalents, qui peuvent le cas échéant contenir des groupements ester, des carbones carbonyle, des groupements amide, des groupements éther et/ou qui peuvent être partiellement fluorés ou perfluorés,
en présence d'un composé de molybdène comme premier composant de catalyseur et de trifluorure de bore, un oxyde d'aluminium, du 1,8-diazobicyclo-[5.4.0]-undéc-7-ène ou du 1,4-diazobicyclo-[2.2.2]-octane comme deuxième composant de catalyseur et
(2.1) alcoxylation de l'hydroxyéther avec un agent d'alcoxylation en un composé amphiphile non ionique ou
(2.2) transformation de l'hydroxyéther en un composé amphiphile ionique (a), le cas échéant avec une alcoxylation préalable (b).

12. Procédé pour la préparation de composés amphiphiles selon la revendication 11, **caractérisé en ce que** la préparation du composé amphiphile ionique (2.2) est réalisée par
transformation de l'hydroxyéther selon l'une quelconque des étapes suivantes :
(a) transformation de l'hydroxyéther avec
du trioxyde de soufre/gaz inerte ; de l'oléum ; de l'acide chlorosulfonique ; de l'acide amidosulfonique ; de l'acide phosphorique ; de l'oxygène en présence d'un dérivé du 2,2',6,6'-tétraméthylpipérinoxyde ; de l'acide halogénoacétique ; une sulfone ; une taurine et/ou de l'anhydride de l'acide maléique, suivie d'hydrogénosulfure de sodium et, le cas échéant, neutralisation
ou
(b) alcoxylation suivie de l'étape (a).

13. Procédé selon l'une quelconque des revendications 11 ou 12 pour la préparation d'un composé amphiphile selon l'une quelconque des revendications 1 à 10.

14. Procédé selon l'une quelconque des revendications 11 à 13, **caractérisé en ce qu'**on réalise la réaction comme une réaction dans un récipient.

15. Procédé selon l'une quelconque des revendications 11 à 14, **caractérisé en ce qu'**on utilise un alcool monovalent ou polyvalent comme solvant.

16. Procédé selon l'une quelconque des revendications 11 à 14, **caractérisé en ce qu'**on travaille à l'abri de l'eau.

17. Procédé selon l'une quelconque des revendications 11 à 14, **caractérisé en ce qu'**on utilise des composés solubles de molybdène.

18. Procédé selon l'une quelconque des revendications 11 à 14, **caractérisé en ce qu'**on utilise du MoO₂(acac)₂ ou du Mo(CO)₆.

19. Procédé selon l'une quelconque des revendications 11 à 14, **caractérisé en ce qu'**on utilise de l'oxyde de molybdène sur un support de catalyseur.

20. Procédé selon la revendication 19, **caractérisé en ce qu'**on utilise comme support de catalyseur des aluminosilicates amorphes ou des zéolithes.

21. Utilisation des composés amphiphiles selon l'une quelconque des revendications 1 à 10 lors de la production d'agents de traitement de plantes, lors d'applications médicales ou lors de la transformation de matériaux synthétiques.
